# EUROPEAN PATENT SPECIFICATION

(11) **EP 3 452 826 B1**
(45) Date of publication and mention of the grant of the patent: **12.02.2020**
(21) Application number: 17713755.1
(22) Date of filing: 17.03.2017
(51) Int. Cl.: A61M 16/00, A61M 16/08, A61M 16/20, A63B 21/00, A63B 23/18, A63B 71/06, G01N 33/497, A61B 5/08

(54) **RESPIRATORY MONITORING APPARATUS**
ATEMÜBERWACHUNGSVORRICHTUNG
APPAREIL DE SURVEILLANCE RESPIRATOIRE

(30) Priority: 07.05.2016 GB 201608128
(43) Date of publication of application: 13.03.2019
(73) Proprietor: Smiths Medical International Limited, Ashford, Kent TN25 4BF (GB)
(72) Inventor: BLOMQUIST, Michael L., Brooklyn Park, Minnesota 55443 (US); BENNETT, Paul James Leslie, Marston Moretaine, Bedfordshire MK43 0AQ (GB); BELISARIO, Anthony Lucio, Luton LU3 4BU (GB)
(74) Representative: Flint, Jonathan McNeill
(86) International application number: PCT/GB2017/000034
(87) International publication number: WO 2017/194906

(56) References cited:
- WO-A1-2015/008013
- US-A1- 2007 265 509
- US-A1- 2014 150 792
- SIMONE SCARLATA ET AL: "Exhaled breath analysis by electronic nose in respiratory diseases", EXPERT REVIEW OF MOLECULAR DIAGNOSTICS, vol. 15, no. 7, 11 May 2015 (2015-05-11), pages 933-956, XP055375157, GB ISSN: 1473-7159, DOI: 10.1586/14737159.2015.1043895

## Description

This invention relates to respiratory monitoring apparatus of the kind including a sensor responsive to a biomarker in exhaled breath.

It is well known that the presence or progress of some diseases can be monitored by sensing various biomarkers in exhaled breath (see for example US 2007/0265509 and SIMONE SCARLATA ET AL: "Exhaled breath analysis by electronic nose in respiratory diseases", EXPERT REVIEW OF MOLECULAR DIAGNOSTICS, vol. 15, no. 7, 11 May 2015 (2015-05-11), pages 933-956, GB ISSN: 1473-7159, DOI: 10.1586/14737159.2015.1043895). Markers indicative of such diseases include hydrogen sulphide, acetone, pentane, toluene ammonia and nitrogen oxides. Various sensing technologies have been proposed, such as, gas chromatography, mass spectroscopy, ion mobility spectrometry and chemiresistive sensors. Diseases that can be monitored from biomarkers in exhaled breath include diabetes, lung cancer, kidney failure, asthma, cystic fibrosis and COPD. One problem with such disease monitoring apparatus is that the breath supplied to the sensor tends to be mainly from the upper part of the bronchial system where the biomarkers may be in low concentrations. Also, normal inspiratory and expiratory breathing may not release very much of the biomarkers.

It is an object of the present invention to provide alternative respiratory apparatus.

According to the present invention there is provided respiratory monitoring apparatus of the above-specified kind, characterised in that the apparatus includes a vibratory expiratory therapy device arranged to provide an alternating resistance to expiratory flow of the user, and that the sensor is located in an expiratory flow path such that it is exposed to vibratory expiratory flow from the user.

The sensor may be selected from a group including chemiresistive sensors, gas chromatography sensors and ion mobility sensors. The vibratory expiratory therapy device may include a rocker arranged to open and close an opening to atmosphere during exhalation. The sensor may be exposed only to expiratory flow and not to inspiratory flow. The sensor may be responsive to a biomarker indicative of a respiratory disease. The sensor may be mounted with a housing of the therapy device. Alternatively, the sensor may be located remotely of the therapy device and be connected with the therapy device via a gas sampling tube. The sensor may also be responsive to substances in the therapy device indicative that the device needs cleaning. The apparatus may be arranged to monitor selectively substances in a part only of the respiratory cycle. The apparatus may include a display arranged to provide a display of detected substance or of a disease associated with the detected substance.

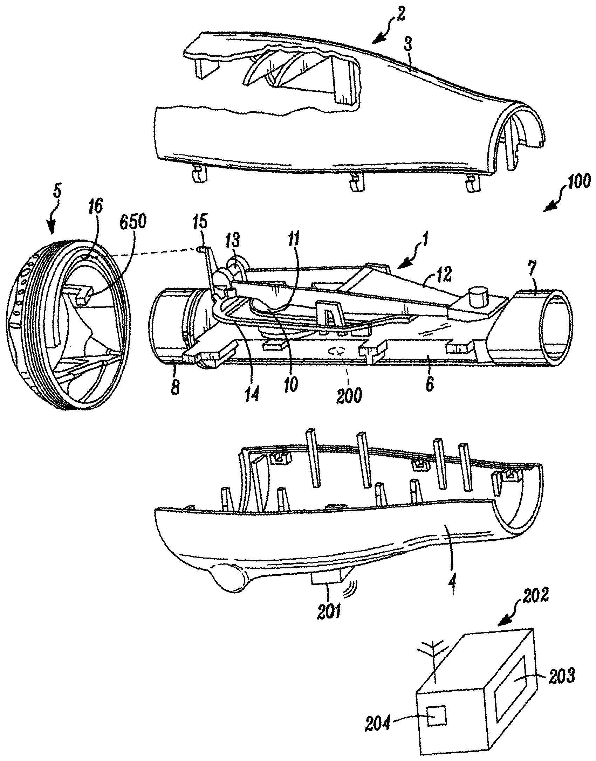

Respiratory monitoring apparatus according to the present invention will now be described, by way of embodiment, with reference to the accompanying drawing which is an exploded view of the apparatus.

The apparatus comprises a respiratory therapy device 100 and a breath sensor 200.

The respiratory therapy device is in the form of a hand-held positive expiratory pressure (PEP) device, that is, a device that presents a resistance to expiration through the device. Such devices are now widely used to help treat patients suffering from a range of respiratory impairments, such as chronic obstructive pulmonary disease, bronchitis, cystic fibrosis and atelectasis. More recently, such devices that provide an alternating resistance to flow have been found to be particularly effective. One example of such a device is sold under the trade mark Acapella (a registered trade mark of Smiths Medical) by Smiths Medical and is described in US6581598, US6776159, US7059324 and US7699054.

The respiratory therapy device 100 comprises a rocker assembly 1 contained within an outer housing 2 provided by an upper part 3 and a lower part 4 of substantially semicylindrical shape. The device is completed by an adjustable dial 5 of circular section. The outer housing 2 contains an air flow tube 6 with a breathing inlet 7 at one end and an inspiratory inlet 8 at the opposite end including a one-way valve (not shown) that allows air to flow into the air flow tube but prevents air flowing out through the inspiratory inlet. The air flow tube 6 has an outlet opening 10 with a non-linear profile that is opened and closed by a conical valve element 11 mounted on a rocker arm 12 pivoted midway along its length about a transverse axis. The air flow tube 6 and housing 2 provide a structure with which the rocker arm 12 is mounted. At its far end, remote from the breathing inlet 7, the rocker arm 12 carries an iron pin 13, which interacts with the magnetic field produced by a permanent magnet (not visible) mounted on an adjustable support frame 14. The magnet arrangement is such that, when the patient is not breathing through the device, the far end of the rocker arm 12 is held down such that its valve element 11 is also held down in sealing engagement with the outlet opening 10. A cam follower projection 15 at one end of the support frame 14 locates in a cam slot 16 in the dial 5 such that, by rotating the dial, the support frame 14, with its magnet, can be moved up or down to alter the strength of the magnetic field interacting with the iron pin 13. The dial 5 enables the frequency of operation and the resistance to flow of air through the device to be adjusted for maximum therapeutic benefit to the user.

When the patient inhales through the breathing inlet 7 air is drawn through the inspiratory inlet 8 and along the air flow tube 6 to the breathing inlet. When the patient exhales, the one-way valve in the inspiratory inlet 8 closes, preventing any air flowing out along this path. Instead, the expiratory pressure is applied via the air flow tube 6 to the underside of the valve element 11 on the rocker arm 12 causing it to be lifted up out of the opening 10 against the magnetic attraction, thereby allowing air to flow out to atmosphere. The opening 10 has a non-linear profile, which causes the effective discharge area to increase as the far end of the rocker arm 12 lifts, thereby allowing the arm to fall back down and close the opening. As long as the user keeps applying sufficient expiratory pressure, the rocker arm 12 will rise and fall repeatedly as the opening 10 is opened and closed, causing a vibratory, alternating or oscillating interruption to expiratory breath flow through the device. Further information about the construction and operation of the device is not essential for an understanding of the invention but can be found in US6581598, US7059324 and US7699054.

The sensor 200 is mounted in the housing 2 where it is exposed to expiratory breath from the patient, such as within the air flow tube 6. In this location it can be seen that the sensor 200 is exposed to both inspiratory and expiratory breath. This may not be a problem. However, it may be that the gas flowing over the sensor 200 during the inspiratory breath (directly from atmosphere) dilutes the responsive of the sensor to the biomarker or other substance of interest. If this is the case, the sensor could be located instead at a point downstream from the valved opening 10, in an extension of the opening, so that the sensor is only exposed to exhaled breaths. The sensor 200 could be of various different types or could include two or more different types of sensor depending on the biomarker being monitored. For example, the sensor could be a gas chromatography sensor, a mass spectroscopy sensor, an ion mobility spectrometer sensor or other type of conventional sensor.

In the present embodiment the sensor 200 is a chemiresistive sensor of the kind including an array of elements of different mixtures of polymer and conductive powder such as carbon. The resistance of each element varies as volatile organic compounds are absorbed by the polymers causing them to expand and increase resistance through the conductive powder. By monitoring the responses of different ones of the elements in the array it is possible to identify the nature of the compound. Further details of such sensors can be seen in US6716638, US6703241, US6627154, US6746960, US6537498, US5571401, US5698089, US5788833, US5911872, US6093308, US6331244, US6010616, US6017440, US5959191, US5951846, US7040139, US7201035, US7819803 and others.

By incorporating the sensor into a vibratory expiratory therapy device the sensor is exposed to an increased proportion of deep lung breath and an increased proportion of biomarkers released more readily from the lung tissue by the vibration produced. A further advantage is that the sensor is exposed to multiple breaths during the progress of the therapy session, thereby enabling more reliable sensing by averaging and or alternatively by integration.

If the sensor is too large or heavy to be contained within the housing of the therapy device it could be located remotely and connected with it via a gas sampling tube, which may require some form of pump to supply a sample of the exhaled gas to the sensor.

The apparatus may include a display of the detected substance or of a disease associated with the detected substance. If the sensor is light enough and small enough it may be possible to provide a display on the housing 2 of the therapy device but, more usually, the output of the sensor 200 is supplied by a unit 201 on the housing, which transmits a signal to a remote processing unit 202 via a cable or by a wireless link, such as by radiofrequency signals with the Bluetooth protocol. The processing unit 202 may have a display 203 and a store 204 for recording the sensor output for subsequent downloading.

The processing unit 202 may have various features. For example, it may be arranged to respond to change in the output of the sensor 200 over time, that is, from session to session, so as to indicate an improvement or deterioration in the user's condition. The processing unit may employ artificial intelligence software to analyse the sensor output and provide an indication of the user's condition. The sensor and processing unit could be arranged to detect whether or not the user has been smoking. The processing unit may be arranged also to receive signals representative of use of the therapy device and provide information showing how the user's condition varies with use of the therapy device. Various upper and lower baselines could be set and information given to the user if these baselines are crossed. Signals could be averaged, filtered or integrated as necessary to provide information in an appropriate form. The sensor and processing unit could be arranged to respond to the presence of specific biomarkers in users with specific diseases, such as the presence of pseudomonas aerugina pathogen in cystic fibrosis patients. The invention is particularly suitable for detecting the respiratory diseases.

The apparatus may have a test feature whereby the sensor is arranged to detect the presence of substances, such as pathogens, inside the therapy device indicating that the device needs cleaning. This could be achieved, for example, by monitoring for the presence of such substances during both inspiratory and expiratory flow through the device. If the pathogen-indicating response is substantially the same during inspiratory and expiratory breaths this is indicative that the pathogen is within the therapy device and not in the user's lungs.

The apparatus could be arranged to detect the presence of airborne pollutants in the lungs that could be harmful to the user, such as benzene and formaldehyde.

The processing unit could be arranged to monitor selectively for substances in a particular part of the respiratory cycle, such as during deep lung expiration only. This could be achieved by selecting a particular part of the waveform of the output from the sensor. Alternatively, the device could have some form of valve arrangement that exposes the sensor to breath flow only during selected parts of the respiratory cycle.

In this application the term "gas" is used to include "vapour".

## Claims

1. Respiratory monitoring apparatus including a sensor (200) responsive to a biomarker in exhaled breath, **characterised in that** the apparatus includes a vibratory expiratory therapy device (100) arranged to provide an alternating resistance to expiratory flow of the user, and that the sensor (200) is located in an expiratory flow path (6) such that it is exposed to vibratory expiratory flow from the user.

2. Apparatus according to Claim 1, **characterised in that** the sensor (200) is selected from a group including chemiresistive sensors, gas chromatography sensors and ion mobility sensors.

3. Apparatus according to Claim 1 or 2, **characterised in that** the vibratory expiratory therapy device (100) includes a rocker (12) arranged to open and close an opening (10) to atmosphere during exhalation.

4. Apparatus according to any one of the claims, **characterised in that** the sensor (200) is exposed only to expiratory flow and not to inspiratory flow.

5. Apparatus according to any one of the preceding claims, **characterised in that** the sensor (200) is responsive to a biomarker indicative of a respiratory disease.

6. Apparatus according to any one of the preceding claims, **characterised in that** sensor (200) is mounted with a housing (2) of the therapy device (100).

7. Apparatus according to any one of Claims 1 to 5, **characterised in that** the sensor is located remotely of the therapy device and is connected with the therapy device via a gas sampling tube.

8. Apparatus according to any one of the preceding claims, **characterised in that** sensor (200) is also responsive to substances in the therapy device (100) indicative that the device needs cleaning.

9. Apparatus according to any one of the preceding claims, **characterised in that** the apparatus is arranged to monitor selectively substances in a part only of the respiratory cycle.

10. Apparatus according to any one of the preceding claims, **characterised in that** the apparatus includes a display (203) arranged to provide a display of detected substance or of a disease associated with the detected substance.

## Patentansprüche

1. Atemüberwachungsvorrichtung mit einem Sensor (200), der auf einen Biomarker in ausgeatmetem Atem anspricht, **dadurch gekennzeichnet, dass** die Vorrichtung eine Vibrationsausatmungstherapieeinrichtung (100) umfasst, die dazu beschaffen ist, einen abwechselnden Widerstand gegen eine Ausatmungsströmung des Benutzers bereitzustellen, und dass der Sensor (200) in einem Ausatmungsströmungspfad (6) angeordnet ist, so dass er der Vibrationsausatmungsströmung vom Benutzer ausgesetzt ist.

2. Vorrichtung nach Anspruch 1, **dadurch gekennzeichnet, dass** der Sensor (200) aus einer Gruppe ausgewählt ist, die chemoresistive Sensoren, Gaschromatographiesensoren und Ionenmobilitätssensoren umfasst.

3. Vorrichtung nach Anspruch 1 oder 2, **dadurch gekennzeichnet, dass** die Vibrationsausatmungstherapieeinrichtung (100) einen Kipphebel (12) umfasst, der dazu beschaffen ist, eine Öffnung (10) zur Atmosphäre während der Ausatmung zu öffnen und zu schließen.

4. Vorrichtung nach einem der Ansprüche, **dadurch gekennzeichnet, dass** der Sensor (200) nur der Ausatmungsströmung und nicht der Einatmungsströmung ausgesetzt ist.

5. Vorrichtung nach einem der vorangehenden Ansprüche, **dadurch gekennzeichnet, dass** der Sensor (200) auf einen Biomarker anspricht, der auf eine Atemerkrankung hinweist.

6. Vorrichtung nach einem der vorangehenden Ansprüche, **dadurch gekennzeichnet, dass** der Sensor (200) an einem Gehäuse (2) der Therapieeinrichtung (100) montiert ist.

7. Vorrichtung nach einem der Ansprüche 1 bis 5, **dadurch gekennzeichnet, dass** der Sensor entfernt von der Therapieeinrichtung angeordnet ist und mit der Therapieeinrichtung über ein Gasprobenrohr verbunden ist.

8. Vorrichtung nach einem der vorangehenden Ansprüche, **dadurch gekennzeichnet, dass** der Sensor (200) auch auf Substanzen in der Therapieeinrichtung (100) anspricht, die darauf hinweisen, dass die Einrichtung eine Reinigung benötigt.

9. Vorrichtung nach einem der vorangehenden Ansprüche, **dadurch gekennzeichnet, dass** die Vorrichtung dazu beschaffen ist, Substanzen in nur einem Teil des Atemzyklus selektiv zu überwachen.

10. Vorrichtung nach einem der vorangehenden Ansprüche, **dadurch gekennzeichnet, dass** die Vorrichtung eine Anzeige (203) umfasst, die dazu beschaffen ist, eine Anzeige einer detektierten Substanz oder einer Erkrankung, die der detektierten Substanz zugeordnet ist, bereitzustellen.

## Revendications

1. Appareil de surveillance respiratoire comprenant un capteur (200) sensible à un biomarqueur dans l'haleine expirée, **caractérisé en ce que** l'appareil comprend un dispositif de thérapie par expiration vibratoire (100) conçu pour fournir une résistance alternative au flux expiratoire de l'utilisateur, et **en ce que** le capteur (200) est situé dans un trajet d'écoulement expiratoire (6) de telle sorte qu'il soit exposé à un flux expiratoire vibratoire de la part de l'utilisateur.

2. Appareil selon la revendication 1, **caractérisé en ce que** le capteur (200) est choisi parmi un groupe comprenant des capteurs à résistance chimique, des capteurs de chromatographie en phase gazeuse et des capteurs de mobilité ionique.

3. Appareil selon la revendication 1 ou 2, **caractérisé en ce que** le dispositif de thérapie par expiration vibratoire (100) comprend un basculeur (12) conçu pour ouvrir et fermer une ouverture (10) à l'atmosphère pendant l'expiration.

4. Appareil selon l'une quelconque des revendications, **caractérisé en ce que** le capteur (200) n'est exposé qu'au flux expiratoire et non au flux inspiratoire.

5. Appareil selon l'une quelconque des revendications précédentes, **caractérisé en ce que** le capteur (200) est sensible à un biomarqueur indicatif d'une maladie respiratoire.

6. Appareil selon l'une quelconque des revendications précédentes, **caractérisé en ce que** le capteur (200) est monté avec un boîtier (2) du dispositif de thérapie (100).

7. Appareil selon l'une quelconque des revendications 1 à 5, **caractérisé en ce que** le capteur est situé à distance du dispositif de thérapie et est connecté au dispositif de thérapie via un tube de prélèvement de gaz.

8. Appareil selon l'une quelconque des revendications précédentes, **caractérisé en ce que** le capteur (200) est également sensible aux substances présentes dans le dispositif de thérapie (100), indiquant que le dispositif doit être nettoyé.

9. Appareil selon l'une quelconque des revendications précédentes, **caractérisé en ce que** l'appareil est conçu pour surveiller sélectivement des substances dans une partie seulement du cycle respiratoire.

10. Appareil selon l'une quelconque des revendications précédentes, **caractérisé en ce que** l'appareil comprend un affichage (203) conçu pour fournir un affichage de substance détectée ou d'une maladie associée à la substance détectée.
